(19) Europäisches Patentamt
European Patent Office
Office européen des brevets

(11) **EP 4 283 622 A1**

(12) **EUROPEAN PATENT APPLICATION**

(43) Date of publication:
**29.11.2023 Bulletin 2023/48**

(21) Application number: **22175179.5**

(22) Date of filing: **24.05.2022**

(51) International Patent Classification (IPC):
**G16H 10/20** (2018.01)      **G16H 50/20** (2018.01)

(52) Cooperative Patent Classification (CPC):
**G16H 10/20; G16H 40/63; G16H 50/50; G16H 50/70**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **SPELT, Hanne, Adriana, Alijda**
  **Eindhoven (NL)**
- **LEUFKENS, Timmy, Robertus, Maria**
  **Eindhoven (NL)**

- **DENISSEN, Adrianus, Johannes, Maria**
  **5656AG Eindhoven (NL)**
- **VAN EE, Raymond**
  **5656AG Eindhoven (NL)**
- **HART DE RUIJTER-BEKKER, Evelijne, Machteld**
  **Eindhoven (NL)**
- **HUIJBERS, Willem**
  **Eindhoven (NL)**
- **VAN DOOREN, Marieke**
  **Eindhoven (NL)**

(74) Representative: **Philips Intellectual Property & Standards**
**High Tech Campus 52**
**5656 AG Eindhoven (NL)**

(54) **COGNITIVE PERFORMANCE DETERMINATION APPARATUS**

(57)      The present invention relates to a cognitive performance determination apparatus (10), comprising:
- an input unit (20); and
- a processing unit (30).

The input unit is configured to provide the processing unit with results of a plurality of trials of a test, wherein the results of each trial is generated by a person undertaking a plurality of events of each trial. The processing unit is configured to determine a plurality of performance values for the results of the plurality of trials of the test, wherein a performance value is determined for each trial of the plurality of trials. The processing unit is configured to determine information about the person, and wherein the determination of the information about the person comprises a calculation of a curve of a model fit to at least some of the plurality of performance values and/or the determination of the information about the person comprises a calculation of performance variability between at least one consecutive pair of performance values.

FIG. 2

EP 4 283 622 A1

**Description**

FIELD OF THE INVENTION

The present invention relates to a cognitive performance determination apparatus, a cognitive performance determination method, as well as to a computer program element and a computer readable medium.

BACKGROUND OF THE INVENTION

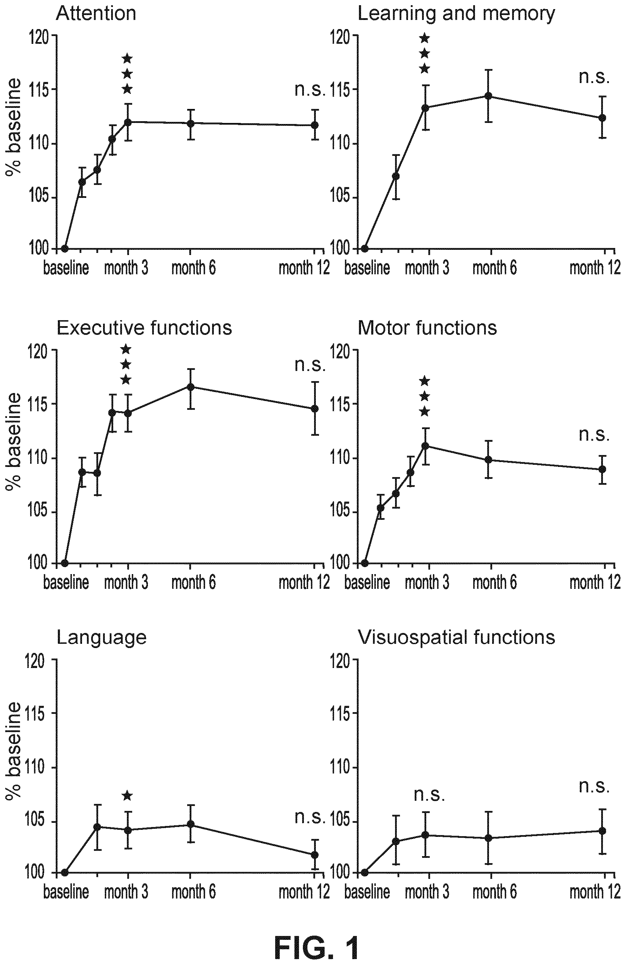

Practice effect with respect to neuropsychological assessments is when a person's performance improves on a task where this is not the primary outcome measure. For some neuropsychological assessments it is recommended to first train and familiarize the person (patient) with a certain cognitive test to ensure that they have reached their performance plateau before the actual assessment of cognitive functioning will take place (see for example: Wesnes, K., & Pincock, C. (2002). Practice effects on cognitive tasks: a major problem?. The Lancet Neurology, 1(8), 473). That way the person (patient) and the assessor can be sure that the person is at their absolute peak and does not show a practice effect in the assessment (or in subsequent assessments), i.e. shows unwanted improvement in a certain cognitive test. A test consists of one or more trials. Each trial consists of a fixed logical order of events, often involving the presentation of a stimulus and processing the response to said stimulus. If a test has more than one trial, the trials can have the same difficulty but not necessarily. A test is for example the Rey Auditory Verbal Learning Test (RAVLT), which consists of 5 trials of repeating words. (Multiple tests are known as a neuropsychological assessment or a test battery).
Practice effects, also referred to as retest or learning effects, are improvements in performance after repeated exposure to test materials (Jutten et al., 2020). Especially in repeated longitudinal assessment this is important, (i.e., Fig. 1 by Bartels, C., Wegrzyn, M., Wiedl, A., Ackermann, V., & Ehrenreich, H. (2010). Practice effects in healthy adults: a longitudinal study on frequent repetitive cognitive testing. BMC neuroscience, 11(1), 1-12). This shows that people score lower on their first test(s) than they should.
Practice effects may lead to underestimating the severity of disease progression or overestimating the efficacy of treatment effects (see for example: Jutten, R. J., Grandoit, E., Foldi, N. S., Sikkes, S. A., Jones, R. N., Choi, S. E., ... & Rabin, L. A. (2020). Lower practice effects as a marker of cognitive performance and dementia risk: a literature review. Alzheimer's & Dementia: Diagnosis, Assessment & Disease Monitoring, 12(1), e12055). There can be various reasons for practice effects; e.g. people might need time to understand the instructions, or to translate the instructions into action, or to reach the peak in their performance.
Pre-training in order that a person (patient) has reached the plateau take valuable time, and it can be difficult to determine when the results of the cognitive test can be properly evaluated.
There is a need to address these issues.

SUMMARY OF THE INVENTION

It would be advantageous to provide an improved technique to determine cognitive performance for a person (patient).
The object of the present invention is solved with the subject matter of the independent claims, wherein further embodiments are incorporated in the dependent claims. It should be noted that the following described aspects and examples of the invention apply to the cognitive performance determination apparatus, the cognitive performance determination method, as well as to a computer program element and a computer readable medium,
**[0009]** In a first aspect, there is provided a cognitive performance determination apparatus, comprising:

- an input unit; and
- a processing unit.

The input unit is configured to provide the processing unit with results of a plurality of trials of a test. The results of each trial is generated by a person undertaking a plurality of events of each trial. The processing unit is configured to determine a plurality of performance values for the results of the plurality of trials of the test. A performance value is determined for each trial of the plurality of trials. The processing unit is configured to determine information about the person. The determination of the information about the person comprises a calculation of a curve of a model fit to at least some of the plurality of performance values and/or the determination of the information about the person comprises a calculation of performance variability between at least one consecutive pair of performance values.
The person can for example be a patient undergoing some form of psychological test. The person can however be someone other than a patient, for example someone undergoing a test for acceptance for further education or a person undergoing a test as part of an application for employment.

In an example, the determination of the information about the person comprises the calculation of performance variability between at least one consecutive pair of performance values. The information about the person can then comprise one or more trials of the plurality of trials selected on the basis of the performance variability between at least one consecutive pair of performance values.

Thus, a test consisting of a number of trials of a fixed logical order of events, often involving the presentation of a stimulus and processing the response to said stimulus, undertaken by the patient will show practice effects as the person's performance gradually increases towards a plateau. Thus, the results of trials that the person has undertaken after a deviation between trails shows that they are now at a plateau in performance can be selected, for example for further analysis by a medical professional/clinician or examination overseer.

In an example, the determination of the information about the person comprises the calculation of performance variability between at least one consecutive pair of performance values. The information about the person can then comprises one or more trials of the plurality of trials that were undertaken by the person after the performance variability between a consecutive pair of performance values is equal to or is below a threshold value.

Thus, as a person carries out trials of a test their performance starts from a baseline and gradually improves towards a plateau that is representative of their actual cognitive capability with respect to the test, and it can then be determined to select those trials at the plateau that are representative for further analysis by a medical professional or test overseer, and not utilise the trial results leading up to the plateau.

In an example, the threshold value is a percentage value and is one of: 1%, 2%, 3%, 4%, 5%, 6%, 7%, 8%, 9%, 10%, 15%, 20%.

In an example, the determination of the information about the person comprises the calculation of performance variability between at least one consecutive pair of performance values. The input unit is configured to provide the processing unit with a performance variability between at least one consecutive pair of performance values for one or more further persons generated from results of a plurality of trials of the test undertaken by the one or more further persons. The information about the person can then comprise a comparison of the performance variability between the at least one consecutive pair of performance values for the person with the performance variability between at least one consecutive pair of performance values for the one or more further persons.

In other words, there variability between trials of a test with respect to the trials leading up to a plateau and of the trials at the plateau can be utilised to provide information on the cognitive ability of the person through comparison of this variability in performance with that of other persons performing the same or similar test.

In an example, the determination of the information about the person comprises the calculation of the curve of the model fit to the at least some of the plurality of performance values. The information about the person can then comprise information derived from the curve of the model fit to the at least some of the plurality of performance values.

Thus, it has been established that model can be fit to the performance improvement of a person as they undertake trials of a test as a performance gradually increases to a plateau. This means that the trials that are at the plateau can be selected for further analysis by medical professional, and the curve itself can be utilised to provide information on the cognitive ability of the person such as through timescale value related to how long it takes the person to reach the plateau. This means, that cognitive information of the person can be determined from trials where the person never actually reaches the plateau, providing for an efficient ability to determine cognitive ability of the person via a minimum number of trials and even for a person for whom it would take a very very long time to reach the plateau.

In an example, the information derived from the curve of the model fit to the at least some of the plurality of performance values comprises one or more trials of the plurality of trials selected on the basis of the curve of the model fit to the at least some of the plurality of performance values.

In other words, the result of the trials at the plateau that are representative of the person's cognitive ability can be selected for further analysis, and those trial results leading up to the plateau can be deselected.

In an example, the information derived from the curve of the model fit to the at least some of the plurality of performance values comprises a time constant of the model used to fit the curve of the model to the at least some of the plurality of performance values.

In other words, a timescale associated with a model fitted to how a person's performance with respect to trials of a test gradually increases provides a new and important additional outcome measure of the neurological assessment that can be provided to a medical professional/clinician or to an exam overseer. To put this another way, the trial results tending toward a plateau, that were previously difficult to utilize in order to help in a neurological assessment, can now be utilized for that neurological assessment via a timescale representative of how the person's cognitive performance increases. This means not only can trial results that were not previously useable be used, but the neurological assessment can be made more accurate.

In an example, the input unit is configured to provide the processing unit with a time constant of the model used to fit the curve of the model to a plurality of performance values for one or more further persons generated from results of a plurality of trials of the test undertaken by the one or more further persons. The information about the person can then comprises a comparison of the time constant of the model used to fit the curve of the model to the at least some of the plurality of performance values for the person with the time constant of the model used to fit the curve of the model to the plurality of performance values for one or more further persons.

Thus, timescale associated with how a person improves with respect to trials of a test towards a steady state plateau can be utilised to provide information on cognitive ability of the person, and one mechanism by which this can be assessed as through comparison to other similar persons carrying out the same or similar tests. This comparison of time constant can be particularly meaningful when comparing between similar persons with respect to age, education, nationality, etc.

In an example, the input unit is configured to provide the processing unit with one or more curves of the model each fit to a plurality of performance values for one or more further persons generated from results of a plurality of trials of the test undertaken by the one or more further persons. The information about the person can then comprise a comparison of the curve of the model fit to the at least some of the plurality of performance values for the person with the one or more curves of the model each fit to a plurality of performance values for one or more further persons.

Thus, a curve fitted to a model of how a person improves with respect to trials of a test towards a steady state plateau can be utilised to provide information on cognitive ability of the patient, and one mechanism by which this can be assessed as through comparison to other patients carrying out the same or similar tests.

In other words, the time constant is an important part of the curve definition that can be utilized for neurological assessment. However, it has been established that the how the curve is fully defined can provide further information. This means that the start performance level and the end plateau height - asymptotic level can in addition to the time constant be used as part of the neurological assessment. Thus, two curves from different persons can have the same or similar time constants but be different with respect to the start level and or plateau height, and this further information can be utilized as part of the neurological assessment.

In an example, the information derived from the curve of the model fit to the at least some of the plurality of performance values comprises an asymptotic performance value of the model used to fit the curve of the model to the at least some of the plurality of performance values. The information about the person can then comprise one or more trials of the plurality of trials that were undertaken by the person after a trial that has a performance value within a threshold value of the asymptotic performance value of the model.

In other words, a curve of a model fit the performance of the person undertaking trials gradually rises towards a plateau in an asymptotic manner, and the asymptotic value associated with this curve indicates the plateau level. This value can then be used for example to indicate value of the performance of the person which itself is an indicator of the cognitive ability of the person which could be compared with the same value for other persons conducting the same or similar test. This value can also be used to select the trial results at the plateau that can be utilised for further analysis, and deselect those trial results leading up to the plateau.

In an example, the determination of the information about the person comprises the calculation of the curve of the model fit to the at least some of the plurality of performance values and the calculation of performance variability between at least one consecutive pair of performance values. The processing unit is configured to determine the at least some of the plurality of performance values as the performance values before a performance value that is one of the consecutive pair performance values that have a performance variability equal to or below a threshold value.

Thus, the variability between consecutive trial results can be utilised to determine when the person has reached a plateau in performance. This can be used to select the trial results prior to this point that are associated with the gradual increase of the person's performance. These trial results leading up to the plateau can then be used to fit the curve of the model, enabling the model to better fit the data and provide more accurate information relating to this model.

In an example, the input unit is configured to provide the processing unit with results of a plurality of trials of a second test. The results of each trial of the second test is generated by the person undertaking a plurality of events of each trial of the second test. The processing unit is configured to determine a plurality of performance values for the results of the plurality of trials of the second test. A performance value is determined for each trial of the plurality of trials of the second test. The processing unit is configured to calculate a curve of the model fit to at least some of the plurality of performance values of the second test and/or to calculate performance variability between at least one consecutive pair of performance values of the second test. The information about the person comprises a comparison of the curve of the model fit to at the least some of the plurality of performance values of the test with the curve of the model fit to at the least some of the plurality of performance values of the second test and/or a comparison of the performance variability between at least one consecutive pair of performance values of the test with the performance variability between at least one consecutive pair of performance values of the second test.

Thus, a person can conduct the same test at different times and the performance of the person taking this test and how it varies can be utilised to determine changes in cognitive ability. Alternatively or additionally, the person can undertake tests associated with different cognitive domains providing for an assessment of different cognitive abilities with respect to different neuropsychological assessments.

In a second aspect, there is provided a cognitive performance determination method, comprising:

providing a processing unit with results of a plurality of trials of a test, wherein the results of each trial is generated by a person undertaking a plurality of events of each trial;

determining by the processing unit a plurality of performance values for the results of the plurality of trials of the test, wherein a performance value is determined for each trial of the plurality of trials; and

determining by the processing unit information about the person, and wherein the determining the information about the person comprises calculating a curve of a model fit to at least some of the plurality of performance values and/or the determining the information about the person comprises calculating performance variability between at least one consecutive pair of performance values.

According to another aspect, there is provided computer program elements controlling one or more of the apparatuses as previously described which, if the computer program element is executed by a processor, is adapted to perform the method as previously described.

According to another aspect, there is provided computer readable media having stored the computer elements as previously described.

The computer program element can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

BRIEF DESCRIPTION OF THE DRAWINGS

Exemplary embodiments will be described in the following with reference to the following drawings:

Fig. 1 (by: Bartels, C., Wegrzyn, M., Wiedl, A., Ackermann, V., & Ehrenreich, H. (2010). Practice effects in healthy adults: a longitudinal study on frequent repetitive cognitive testing. BMC neuroscience, 11(1), 1-12) shows examples of practice effects;

Fig. 2 shows a schematic representation of a cognitive performance determination apparatus;

Fig. 3 shows a cognitive performance determination method;

Fig. 4 shows an example of a person's performance with respect to taking trials of a test;

Fig. 5 shows an example of a person's performance with respect to taking trials of a test;

Fig. 6 shows an example of a person's performance with respect to taking trials of a test;

Fig. 7 shows an example of a person's performance with respect to taking trials of a test; and

Fig. 8 shows an example of the elementary building blocks of the new technique.

DETAILED DESCRIPTION OF EMBODIMENTS

The new technique provides an ability to aid performance assessment in the context of neuropsychological assessments, however the technique is also applicable to other cognitive or motor function assessments (e.g. SAT). Thus, such test can also be referred to as cognitive tests.

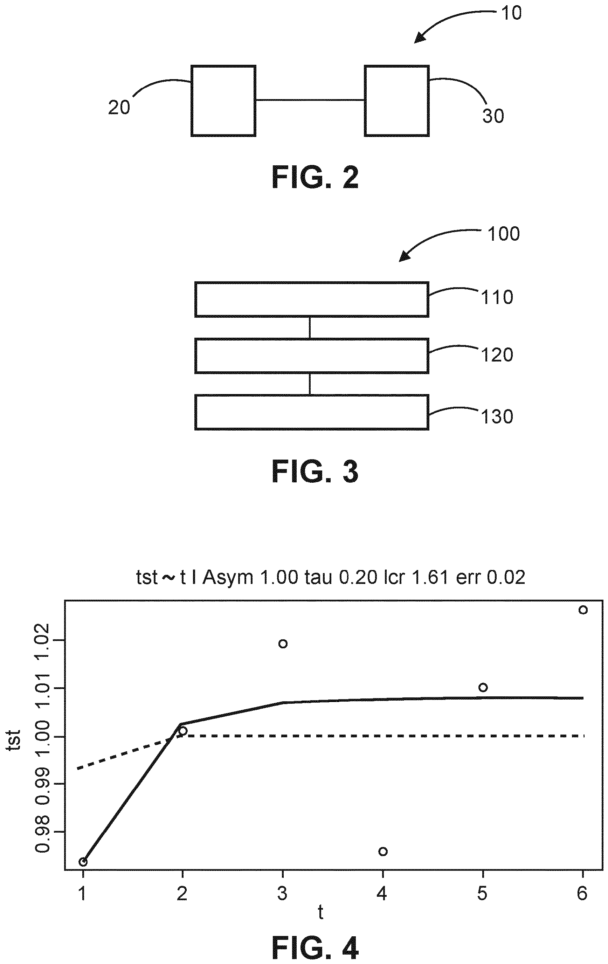

Fig. 2 shows an example of a cognitive performance determination apparatus 10. The apparatus comprises an input unit 20, and a processing unit 30. The input unit is configured to provide the processing unit with results of a plurality of trials of a test, and the results of each trial is generated by a person undertaking a plurality of events of each trial. The processing unit is configured to determine a plurality of performance values for the results of the plurality of trials of the test, and a performance value is determined for each trial of the plurality of trials. The processing unit is configured to determine information about the person. The determination of the information about the person comprises a calculation of a curve of a model fit to at least some of the plurality of performance values. Additionally or alternatively the determination of the information about the person comprises a calculation of performance variability between at least one consecutive pair of performance values.

According to an example, the determination of the information about the person comprises the calculation of performance variability between at least one consecutive pair of performance values. The information about the person can then comprise one or more trials of the plurality of trials selected on the basis of the performance variability between at least one consecutive pair of performance values.

According to an example, the determination of the information about the person comprises the calculation of performance variability between at least one consecutive pair of performance values. The information about the person can then comprise one or more trials of the plurality of trials that were undertaken by the person after the performance variability between a consecutive pair of performance values is equal to or is below a threshold value.

According to an example, the threshold value is a percentage value and is one of: 1%, 2%, 3%, 4%, 5%, 6%, 7%, 8%, 9%, 10%, 15%, 20%.

In an example, the input unit is configured to provide the processing unit with the threshold value input from a user.

Thus, the default threshold value of for example 5% can be utilised to determine when the plateau is reached when the variability between consecutive trials is 5% or less, and a medical professional can adjust this if necessary based on their experience.

According to an example, the determination of the information about the person comprises the calculation of performance variability between at least one consecutive pair of performance values. The input unit is configured to provide the processing unit with a performance variability between at least one consecutive pair of performance values for one or more further persons generated from results of a plurality of trials of the test undertaken by the one or more further persons. The information about the person can then comprise a comparison of the performance variability between the at least one consecutive pair of performance values for the person with the performance variability between at least one consecutive pair of performance values for the one or more further persons.

According to an example, the determination of the information about the person comprises the calculation of the curve of the model fit to the at least some of the plurality of performance values. The information about the person can then comprise information derived from the curve of the model fit to the at least some of the plurality of performance values.

According to an example, the information derived from the curve of the model fit to the at least some of the plurality of performance values comprises one or more trials of the plurality of trials selected on the basis of the curve of the model fit to the at least some of the plurality of performance values.

According to an example, the information derived from the curve of the model fit to the at least some of the plurality of performance values comprises a time constant of the model used to fit the curve of the model to the at least some of the plurality of performance values.

According to an example, the input unit is configured to provide the processing unit with a time constant of the model used to fit the curve of the model to a plurality of performance values for one or more further persons generated from results of a plurality of trials of the test undertaken by the one or more further persons. The information about the person can then comprise a comparison of the time constant of the model used to fit the curve of the model to the at least some of the plurality of performance values for the person with the time constant of the model used to fit the curve of the model to the plurality of performance values for one or more further persons.

According to an example, the input unit is configured to provide the processing unit with one or more curves of the model each fit to a plurality of performance values for one or more further persons generated from results of a plurality of trials of the test undertaken by the one or more further persons. The information about the person can then comprise a comparison of the curve of the model fit to the at least some of the plurality of performance values for the person with the one or more curves of the model each fit to a plurality of performance values for one or more further persons.

According to an example, the information derived from the curve of the model fit to the at least some of the plurality of performance values comprises an asymptotic performance value of the model used to fit the curve of the model to the at least some of the plurality of performance values. The information about the person can then comprise one or more trials of the plurality of trials that were undertaken by the person after a trial that has a performance value within a threshold value of the asymptotic performance value of the model.

In an example, the threshold value is a percentage value and is one of: 1%, 2%, 3%, 4%, 5%, 6%, 7%, 8%, 9%, 10%, 15%, 20%.

In an example, the input unit is configured to provide the processing unit with the threshold value input from a user.

It however to be noted that the performance leading up to the plateau can be utilized for neurological assessment and thus those trials at the plateau need not be utilized and indeed the test may not need to proceed until the trail results indicate that the person has reached the plateau, rather just the trial results during the increase in performance phase can be utilized for the neurological assessment, through for example a determination of a characteristic time constant associated with the increase in performance.

According to an example, the determination of the information about the person comprises the calculation of the curve of the model fit to the at least some of the plurality of performance values and the calculation of performance variability between at least one consecutive pair of performance values. The processing unit is configured to determine the at least some of the plurality of performance values as the performance values before a performance value that is one of the consecutive pair performance values that have a performance variability equal to or below a threshold value.

In an example, the threshold value is a percentage value and is one of: 1%, 2%, 3%, 4%, 5%, 6%, 7%, 8%, 9%, 10%, 15%, 20%.

In an example, the input unit is configured to provide the processing unit with the threshold value input from a user.

According to an example, the input unit is configured to provide the processing unit with results of a plurality of trials of a second test, and the results of each trial of the second test is generated by the person undertaking a plurality of events of each trial of the second test. The processing unit is configured to determine a plurality of performance values for the results of the plurality of trials of the second test, and a performance value is determined for each trial of the plurality of trials of the second test. The processing unit is configured to calculate a curve of the model fit to at least some of the plurality of performance values of the second test and/or to calculate performance variability between at least one consecutive pair of performance values of the second test. The information about the person can then comprise a comparison of the curve of the model fit to at the least some of the plurality of performance values of the test with the curve of the model fit to at the least some of the plurality of performance values of the second test and/or a comparison of the performance variability between at least one consecutive pair of performance values of the test with the performance variability between at least one consecutive pair of performance values of the second test.

In an example, the second test is the same test as the test.

In an example, the second test is a different test to the test.

In an example, the test relates to a first cognitive domain and the second test relates to a second cognitive domain.

Fig. 3 shows a cognitive performance determination method 100, comprising:

providing 110 a processing unit with results of a plurality of trials of a test, wherein the results of each trial is generated by a person undertaking a plurality of events of each trial;

determining 120 by the processing unit a plurality of performance values for the results of the plurality of trials of the test, wherein a performance value is determined for each trial of the plurality of trials; and

determining 130 by the processing unit information about the person, and wherein the determining the information about the person comprises calculating a curve of a model fit to at least some of the plurality of performance values and/or the determining the information about the person comprises calculating performance variability between at least one consecutive pair of performance values.

In an example, the determining the information about the person comprises the calculating performance variability between at least one consecutive pair of performance values, and wherein the information about the person comprises one or more trials of the plurality of trials selected on the basis of the performance variability between at least one consecutive pair of performance values.

In an example, the determining the information about the person comprises the calculating performance variability between at least one consecutive pair of performance values, and wherein the information about the person comprises one or more trials of the plurality of trials that were undertaken by the person after the performance variability between a consecutive pair of performance values is equal to or is below a threshold value.

In an example, the threshold value is a percentage value and is one of: 1%, 2%, 3%, 4%, 5%, 6%, 7%, 8%, 9%, 10%, 15%, 20%.

In an example, the method comprises providing the processing unit with the threshold value input from a user.

In an example, the determining the information about the person comprises the calculating the curve of the model fit to the at least some of the plurality of performance values and the calculating performance variability between at least one consecutive pair of performance values, wherein the method comprises providing the processing unit with a performance variability between at least one consecutive pair of performance values for one or more further persons generated from results of a plurality of trials of the test undertaken by the one or more further persons, and wherein the information about the person comprises comparing the performance variability between the at least one consecutive pair of performance values for the person with the performance variability between at least one consecutive pair of performance values for the one or more further persons.

In an example, the determining the information about the person comprises the calculating the curve of the model fit to the at least some of the plurality of performance values, and wherein the information about the person comprises information derived from the curve of the model fit to the at least some of the plurality of performance values.

In an example, the information derived from the curve of the model fit to the at least some of the plurality of performance values comprises one or more trials of the plurality of trials selected on the basis of the curve of the model fit to the at least some of the plurality of performance values.

In an example, the information derived from the curve of the model fit to the at least some of the plurality of performance values comprises a time constant of the model used to fit the curve of the model to the at least some of the plurality of performance values.

In an example, the method comprises providing the processing unit with a time constant of the model used to fit the curve of the model to a plurality of performance values for one or more further persons generated from results of a plurality of trials of the test undertaken by the one or more further persons, and wherein the information about the person comprises comparing the time constant of the model used to fit the curve of the model to the at least some of the plurality of performance values for the person with the time constant of the model used to fit the curve of the model to the plurality of performance values for one or more further persons.

In an example, the method comprises providing the processing unit with one or more curves of the model each fit to a plurality of performance values for one or more further persons generated from results of a plurality of trials of the test undertaken by the one or more further persons, and wherein the information about the person comprises comparing the curve of the model fit to the at least some of the plurality of performance values for the person with the one or more curves of the model each fit to a plurality of performance values for one or more further persons.

In an example, the information derived from the curve of the model fit to the at least some of the plurality of performance values comprises an asymptotic performance value of the model used to fit the curve of the model to the at least some of the plurality of performance values, and wherein the information about the person comprises one or more trials of the plurality of trials that were undertaken by the person after a trial that has a performance value within a threshold value of the asymptotic performance value of the model.

In an example, the threshold value is a percentage value and is one of: 1%, 2%, 3%, 4%, 5%, 6%, 7%, 8%, 9%, 10%, 15%, 20%.

In an example, the input unit is configured to provide the processing unit with the threshold value input from a user.

In an example, the determining the information about the person comprises the calculating the performance variability between at least one consecutive pair of performance values, and wherein the method comprises determining by the processing unit the at least some of the plurality of performance values as the performance values before a performance value that is one of the consecutive pair performance values that have a performance variability equal to or below a threshold value.

In an example, the threshold value is a percentage value and is one of: 1%, 2%, 3%, 4%, 5%, 6%, 7%, 8%, 9%, 10%, 15%, 20%.

In an example, the method comprises providing the processing unit with the threshold value input from a user.

In an example, the method comprises providing the processing unit with results of a plurality of trials of a second test, wherein the results of each trial of the second test is generated by the person undertaking a plurality of events of each trial of the second test; wherein the method comprises determining by the processing unit a plurality of performance values for the results of the plurality of trials of the second test, wherein a performance value is determined for each trial of the plurality of trials of the second test, wherein the method comprises calculating by the processing unit a curve of the model fit to at least some of the plurality of performance values of the second test and/or calculating by the processing unit performance variability between at least one consecutive pair of performance values of the second test, and wherein the information about the person comprises comparing the curve of the model fit to at the least some of the plurality of performance values of the test with the curve of the model fit to at the least some of the plurality of performance values of the second test and/or comparing the performance variability between at least one consecutive pair of performance values of the test with the performance variability between at least one consecutive pair of performance values of the second test.

In an example, the second test is the same test as the test.

In an example, the second test is a different test to the test.

In an example, the test relates to a first cognitive domain and the second test relates to a second cognitive domain.

Thus, as pre-training takes valuable time, the new technique has been developed that for example has the benefit to estimate the plateau of the performance and time to reach it without executing a very large number of trials.

Thus, the new technique detects a performance plateau during neurophysiological assessments using performance data. This can be used to quantify practice effects and interpret these by comparing to a normalized average, longitudinal assessments or between cognitive domains. This leads to several advantages:

More reliable and better results by considering only datapoints when a performance plateau is reached.

Increased sensitivity in testing as time to reach performance plateau can serve as additional measure in a neuropsychological assessment.

The cognitive performance determination apparatus and the cognitive performance determination method are now described in further specific detail, where reference is made to Figs, 4-8.

Typically practice effects are viewed as a source of bias in repeated neuropsychological assessments.

However, it was realized that they can also provide additional information about the person's cognitive capabilities, and this led to the development of the new cognitive performance determination apparatus and the cognitive performance determination method described here.

Thus, if a person takes longer to reach a performance plateau this may be an early indication of a change in cognitive functioning. In disorders where learning is compromised, such as Alzheimer's disease (AD) or mild cognitive impairment (MCI), attenuated practice effects can be expected. In general or on specific tasks - e.g., lower practice effects on episodic memory tasks have been related to AD dementia (see for example: Jutten, R. J., Grandoit, E., Foldi, N. S., Sikkes, S. A., Jones, R. N., Choi, S. E., ... & Rabin, L. A. (2020). Lower practice effects as a marker of cognitive performance and dementia risk: a literature review. Alzheimer's & Dementia: Diagnosis, Assessment & Disease Monitoring, 12(1), e12055). In other words; variability in practice effects can be used to define cases or predict longitudinal outcomes.

Thus, in the new technique described here providing a comparison of learning curves to a normalized average, between longitudinal assessments, or between cognitive domains can therefore provide important additional information on the change in a person's neuropsychology. Therefore, a method to detect, quantify and compare these practice effects was developed to enable these beneficial effects.

Typically, each neuropsychological assessment (or test battery) consists of number of neuropsychological tests (e.g. RAVLT, TMT, etc.). In each test the person is presented with a number of items and a logical sequence of (presenting and processing responses to) items is called a trial. Some tests can have only one trial. An assumption was made in the new development that after each trial the next one will be executed faster or with less errors. It was then taken that this general learning process will continue until hardly any improvement is observable anymore: the performance plateau is reached. It was then determined in the new technique that this general learning process can be described with the following model formula:

$$SSasymp(t) = Asym + (Perf0 - Asym) * \exp(-t/tau)$$

Where:

$t$ = a numeric vector that represents trial number 1, 2,..

*Asym* = a numeric parameter representing the upper asymptotic value of the model

*Perf0* = a numeric parameter representing the performance at t=0 (i.e., before training)

*tau* = a numeric parameter representing the learning constant

The relative error between succeeding trials can be defined by the following formula:

$$rel.\, error[i] = 2 * \frac{Perf[i] - Perf[i-1]}{Perf[i] + Perf[i-1]}$$

Applying these two formulas to trial data the performance plateau and the time to reach it can be estimated without executing a very large number of training sessions.

For people with a small *tau* - reaching their performance plateau after only a few trials - the relative error between two succeeding training trails can be sufficient to determine their plateau. That is if the difference in performance on trial *i* vs trial *i-l* is smaller than for instance 5% the person has reached a plateau in performance. There can be multiple explanations for a small tau including pre (at home) training, having done a similar assessment recently, or high cognitive function.

For people with a larger *tau* - reaching their performance plateau after >3 trials - a plateau in performance can be estimated by fitting a non-linear model to the performance data (see for example: Venables, W. N., & Ripley, B. D. (2003). Modern Applied Statistics With S. (J. Chambers, W. Eddy, W. Härdle, S. Sheather, & L. Tierney, Eds.), Technometrics (Fourth Edi, Vol. 45). New York, NY Springer Science+Business Media). Considering the 3 parameters of the model formula equation above, data of at least 4 trails are needed. Each additional trial will make the estimate more reliable.

Thus, in a specific embodiment of the new technique the first step is assessing the patient's variability between consecutive trials for <3 trials. If the variability between these trials is smaller than for instance 5% it can be safely assumed that the patient is on their performance plateau. Thus, fitting the performance data to a curve is not needed. However, if the variability in the first 3 trials is larger than for instance 5% more trials are needed, and the patient's performance should be fitted to a general learning curve to estimate plateau. Using these two formulas the performance plateau can be estimated regardless of any pretraining of the patient. It is to be noted that a threshold of 5% can be set initially, as this is a common threshold used in statistics, and the clinician can re-evaluate this threshold at her/his own discretion and increase it or reduce it as necessary.

It is to be noted that by fitting the performance data to a curve the plateau can be predicted before it is reached. This feature can be especially valuable for patients that need a very large number of trials to reach their plateau. In this case a reliable estimated of learning ability and performance can be provided without the need to complete all trials. Thus, a large amount of time is saved. Also, by fitting the performance data to a curve, differences in the learning curve between patients or for the same patient over time can be detected, where the difference in the curves can be represented by differences in tau. This difference in tau can be interpreted using contextual knowledge such as time between testing, degeneration on MRI niveau, age, and task complexity.

Figs. 4-7 then show some illustrative examples, showing cases that differ in terms of how fast the person learns

(*tau:* that is the time to reach the plateau). In the figures *tst* stands for performance and t for the number of trials.

In Fig. 4, this person reached a plateau in performance after 2 trials because the relative error between trial 1 and 2 is smaller than 5% (2.8%).

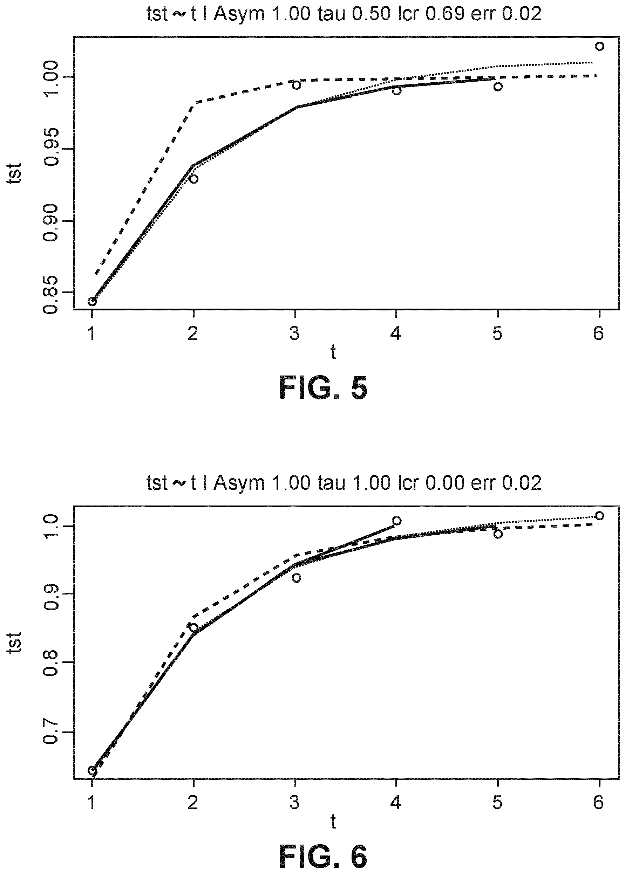

In Fig. 5, this person reached a plateau in performance after 4 trials, because the relative error between trial 3 and 4 is dropped below 5% (0.39%). Because the person already executed 4 trials a non-linear fit with the learning model can be performed to give an estimations and error margin of the asymptotic value ($p < 0.05$), *tau* ($p > 0.1$) and response at time 0 ($p > 0.1$).

In Fig, this person reached a plateau in performance after 5 g trials, because the relative performance dropped below 5% between trial 4 and 5 (2.0%). The non-linear model can use now 5 data points to give more accurate parameter estimations.

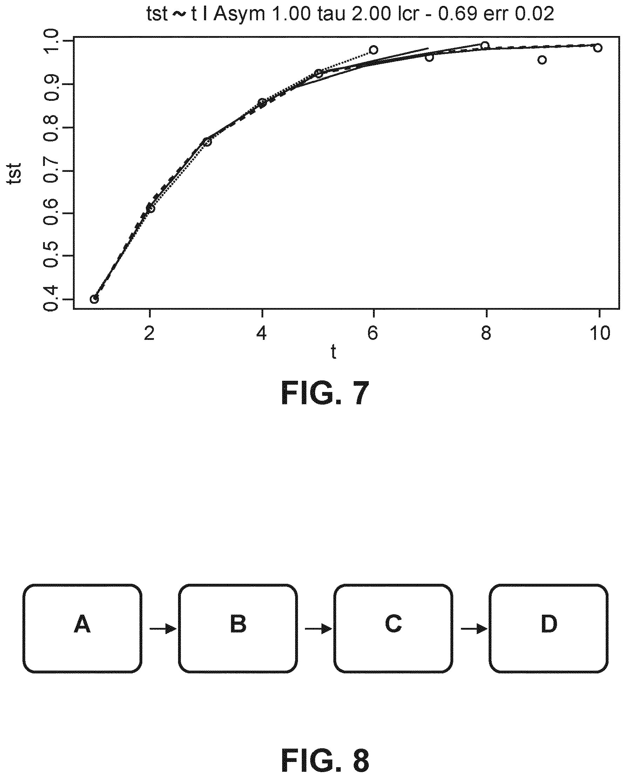

In Fig. 7, this person reached a plateau in performance after 7 trials, because the relative performance dropped below 5% between trial 6 and 7 (1.7%). The non-linear model can use now 7 data points to give more accurate parameter estimations.

As discussed above, the new technique involves 1. fitting performance data to a general learning curve, and/or 2. considering the relative error between trials as an indicator of performance plateau.

Here, performance can be fitted to a general learning curve, one neuropsychological assessment can consist of one or multiple tests, with each test consisting of multiple trials, and here a longitudinal assessment is comparing assessments over time.

In a complete system that makes use of the new technique (the cognitive performance determination apparatus and the cognitive performance determination method), shown schematically in Fig. 8. In Fig. 8 the first box A represents "presentation" with an interface that presents sensory training stimuli to a patient, the second box B represents "Acquisition" to acquire performance data, the third box C represents "Processing" with a processor to execute the described new cognitive performance determination apparatus and a cognitive performance determination method/algorithm related to the performance points and plateau determination, and box D represents "Recommendation" with a unit to log/store or present data related to reaching the plateau.

Thus, in such an exemplar complete system the following can be utilized:

An interface/unit that presents sensory training stimuli to a patient.
Measuring/sensor unit to acquire performance data;
A processor to execute an algorithm that
Receives data from the measuring unit.
Optionally, receives the minimum and maximum performance score for that test.
Determines the occurrence of a performance plateau, which is when

The relative error between the first and the second trial is smaller than for instance 5% using

$$rel.\,error[i] = 2 * \frac{Perf[i] - Perf[i-1]}{Perf[i] + Perf[i-1]}$$

OR when after 4 or more trials the non-linear model estimates learning parameters *Asym, Per0* and *tau,* using

$$SSasymp(t) = Asym + (Perf0 - Asym) * \exp(-t/tau)$$

and stops when last performance was within for instance 5% of estimated *Asym*

Then, training stops automatically, and the last trials (close enough to the plateau) count for the assessment or the learning model is used to correct for the missed performance in the previous trials. This correction relates to the case of someone that practiced the test before or a set up in which data for a specific trial is missed/lost. Using the model we can determine that a person has encountered the test before, because their start performance (Perf0 in the model) is too high and in effect an estimation can be made of the trials with missing data as the curve would pass through the associated performance points for such missing data.

Unit to log/store or present data related the occurrence of a performance plateau.

The following describes the cognitive performance determination apparatus and the cognitive performance determination method via a discussion of several exemplar embodiments.

**First Embodiment**

The first embodiment can be used to detect a learning plateau and quantify the practice effect during a neuropsychological test. This embodiment has elements as described above. Preferably the assessment is giving on a digital platform, such as IntelliSpace Cognition. This embodiment can be applied to any type of neuropsychological test as long as the performance data can be captured and processed during or immediately after the test (e.g. the SDMT, Star Cancelation, STROOP) and the test consists of multiple trials (or enough versions of the test with the same difficulty level are available). The performance data is to be processed immediately after the trial is finished. Alternatively, the performance data is available for processing in real-time (then performance data may need to be normalized or it may be beneficial to use an average performance over a set time, or an average within a moving window). A performance plateau is detected using the formulas as described above. Optionally, a display unit to show the measured and processed signals to the assessor, indicates when a performance plateau is reached or stops the training. The last trial is within the plateau and can optionally count for the assessment.

**Second Embodiment**

The second embodiment can consist of all elements of the first embodiment and additionally compares the time it takes to reach a plateau in performance on a particular test between consecutive assessments of a person (i.e. longitudinal assessment). This comparison informs on how good the person (patient) holds on to the capabilities to perform that test. The relevant parameter here is tau which is a numeric parameter representing the learning constant. When a test is new, a person needs time to learn it - so the first assessment has the largest tau. Any time after that it is expected that tau is shorter, except if the time between the two assessment is long enough to unlearn the test (this depends on e.g., the type of test and characteristics of the respondent). If during an assessment the tau is similar or longer than in the preceding assessment, this may indicate a deterioration in a part of cognitive function. Optionally one may correct this comparison for the time between tests (parts of the test are more likely to be remembered when the time between tests is shorter than longer, which affect the practice effect). Optionally, one may inspect the comparison of learning curves between assessment for one domain and contrast this to a second domain. This comparison of learning curve between assessments can provide valuable insight on the cognitive ability of the person, in general or for specific cognitive domains. Here, reference to domains refers to cognitive domains. Domains can for example be 'Working Memory' or Executive Function'. Their definition depends upon the model that is used. (see for example: S. Vermeent et al: Evidence of Validity for a Newly Developed Digital Cognitive Test Battery, Frontiers in Psychology, 11, 2020, and S. Vermeent et al: Philips Intellispace Cognition digital test battery: equivalence and measurement invariance compared to traditional analog test versions, The clinical Neuropsychologist 2021.). Each domain can be assessed with specific tests. Therefore to get an overview of someone's cognitive abilities, neuropsychological assessments consist of multiple tests assessing several cognitive domains.

Thus, a comparison between curves of the model for the patient doing the same test at different times. It is common practice that one patient does a neuropsychological assessment (NPA) consisting of the same test several times in their lifetime. During each NPA using the new technique a general tau can be calculated - that is an average learning curve for all tests in the NPA. Also, a domain-specific tau can be calculated - that is an average learning curve for a set of tests that correspond to a cognitive domain. Also a test specific tau can be calculated - that is a tau for a specific test. These three types of taus can then be compared between the neuropsychological assessments that a patient completed in their life to provide information on for example cognitive impairment or decline.

**Third embodiment**

The third embodiment can consist of all elements of the first or second embodiment and additionally compares the practice effects on each test in an assessment to a normalized population average. Specifically, tau is compared to taus of similar cases. If a tau is considerably larger than the population average - for the whole assessment, between parts of the assessment (beginning vs ending), on a specific group of tests, or on a specific test - but performance is within a normal range, the deviation of tau may hint at an issue. E.g. but not limited to:

- For the whole assessment or between parts of the assessment (e.g. beginning vs ending): If a person is generally slower, this can provide information about procedural learning, for example, or translating the instructions into an action. This kind of information can be valuable to the assessor.
- On a specific group of tests representing a particular cognitive domain: Domains are for example working memory, processing speed, verbal processing, executive functioning, visual spatial processing, etc. This comparison can give an early indication of cognitive deficiencies on that domain which might be linked to a particular diagnosis.
- On a specific test: If a person has a larger tau than average on a particular test this could for example hint at a specific abnormality in an underlying cognitive function, or incorrect assessment of that test.

Any of these deviations of tau to a normalized population average may be utilized to make a reference for further inspection.

## Fourth Embodiment

The fourth embodiment is consistent with all previous embodiments. In this embodiment an estimate of performance in the beginning of the task (t=0), is used to detect if a patient had prior experience with the task (prior learning). When the model can estimate a learning curve reliably, performance at the intercept (t=0 - Here Perf0 as one of the model parameters) is contrasted with a distribution of performance estimated from task-naive patients. When the estimate at t=0, is more than one standard deviation above the norm, the test instance can be marked as "likely had prior experience". This warning can be used in data analyses to correct results or provide context. Also, it could trigger actions during the test battery. In the SDMT, this warning could trigger actions in the clinical workflow. E.g. suggest to the test administer to ask a question: "Do you use an application to train your cognition?". Similarly, for the RAVLT, if performance at t=0 is unreasonably high, a different question is suggested: "Did you see this list of words during an earlier visit?" The normed estimate of performance at t=0 can be corrected for demographic (e.g. age, gender, education) and/or clinical factors. In case of test that are designed for longitudinal administration (multiple tests), a distinct norm distribution could be used at visit 2, to detect if patients have started training on a task between visit 1 and 2. Besides the intercept at t=0, other metrics to estimate prior learning can be used or combined to estimate prior experience with a task. For example, the first derivate/slope over the initial trials, task-specific parameters related to response times, or the amount of time spend listening to instructions or on practice trials. In certain tests response time is part of the assessment, especially tasks that target the cognitive domain 'processing speed' such as Trail-Making Test A, or the Stroop color-naming test, and here the specific parameters that relate to response time differ per test.

A number of acronyms have been used above, details of these are as follows:

SAT: Scholastic Assessment/Aptitude Test; a standardized test widely used for college admissions in the US.
RAVLT; Rey Auditory Verbal Learning Test (RAVLT); a neuropsychological test assessing (Working/Learning) Memory. Rey Auditory Verbal Learning Test.docx (live.com)
TMT: Trail Making Test is a neuropsychological test assessing working memory and executive functioning. It consists of two parts: in part A the patient Is asked to connect 25 circled numbers in numerical order, in part B the patient connects 25 encircled numbers and letters in numerical and alphabetical order alternating.

Star cancellation: is a neuropsychological test developed to detect the presence of unilateral spatial neglect. A participant is required to search for and cross out ("cancel") targets, which are usually embedded among distractor stimuli. The number of cancelled targets and their location can be used to diagnose the neglect syndrome after stroke.

STROOP: The Stroop task is a neuropsychological test assessing executive functioning and processing speed. It requires individuals to view a list of words that are printed in a different color than the meaning of the word. Participants are tasked with naming the color of the word, not the word itself, as fast as they can.

SDMT: Symbol Digit Modalities test is a neuropsychological test assesses neurological dysfunction. A patient is given a key of numbers corresponding to a set of figures. They then are asked to 'translate' a set of figures by assigning the correct number to each figure using the key.

PPG: Photoplethysmography (PPG) is an uncomplicated and inexpensive optical measurement method that is often used for heart rate monitoring purposes. It measures pulse using reflection of a red or green light.

EDA sensor: Electrodermal activity (EDA) refers to skin conductance or sweating. It is often assessed using two biosignals SCL and number of SCRs.

SCL: Skin conductance level is the tonic level of skin conductance.

Number of SCRs: Skin conductance responses are tiny phasic components on top of the slowly moving skin conductance level. The number of SCRs within a certain timeframe can be related to sympathetic nervous system activity.

Decrease in HRV: HRV stands for Heart Rate Variability. This is a biosignal. It refers to the time between two consecutive heart beats. The variability in these timings relate to nervous system activity (less time between beats - low HRV - relates to more sympathetic arousal).

In another exemplary embodiment, a computer program or computer program element is provided that is characterized by being configured to execute the method steps of any of the methods according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above described system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. A cognitive performance determination apparatus (10), comprising:

   - an input unit (20); and
   - a processing unit (30);
   wherein the input unit is configured to provide the processing unit with results of a plurality of trials of a test, wherein the results of each trial is generated by a person undertaking a plurality of events of each trial;
   wherein the processing unit is configured to determine a plurality of performance values for the results of the plurality of trials of the test, wherein a performance value is determined for each trial of the plurality of trials; and
   wherein the processing unit is configured to determine information about the person, and wherein the determination of the information about the person comprises a calculation of a curve of a model fit to at least some of the plurality of performance values and/or the determination of the information about the person comprises a calculation of performance variability between at least one consecutive pair of performance values.

2. Apparatus according to claim 1, wherein the determination of the information about the person comprises the calculation of performance variability between at least one consecutive pair of performance values, and wherein the information about the person comprises one or more trials of the plurality of trials selected on the basis of the performance variability between at least one consecutive pair of performance values.

3. Apparatus according to any of claims 1-2, wherein the determination of the information about the person comprises the calculation of performance variability between at least one consecutive pair of performance values, and wherein the information about the person comprises one or more trials of the plurality of trials that were undertaken by the person after the performance variability between a consecutive pair of performance values is equal to or is below a threshold value.

4. Apparatus according to claim 3, wherein the threshold value is a percentage value and is one of: 1%, 2%, 3%, 4%, 5%, 6%, 7%, 8%, 9%, 10%, 15%, 20%.

5. Apparatus according to any of claims 1-4, wherein the determination of the information about the person comprises the calculation of performance variability between at least one consecutive pair of performance values, wherein the input unit is configured to provide the processing unit with a performance variability between at least one consecutive pair of performance values for one or more further persons generated from results of a plurality of trials of the test undertaken by the one or more further persons, and wherein the information about the person comprises a comparison of the performance variability between the at least one consecutive pair of performance values for the person with the performance variability between at least one consecutive pair of performance values for the one or more further persons.

6. Apparatus according to any of claims 1-5, wherein the determination of the information about the person comprises the calculation of the curve of the model fit to the at least some of the plurality of performance values, and wherein the information about the person comprises information derived from the curve of the model fit to the at least some of the plurality of performance values.

7. Apparatus according to claim 6, wherein the information derived from the curve of the model fit to the at least some of the plurality of performance values comprises one or more trials of the plurality of trials selected on the basis of the curve of the model fit to the at least some of the plurality of performance values.

8. Apparatus according to any of claims 6-7, wherein the information derived from the curve of the model fit to the at least some of the plurality of performance values comprises a time constant of the model used to fit the curve of the model to the at least some of the plurality of performance values.

9. Apparatus according to claim 8, wherein the input unit is configured to provide the processing unit with a time constant of the model used to fit the curve of the model to a plurality of performance values for one or more further persons generated from results of a plurality of trials of the test undertaken by the one or more further persons, and wherein the information about the person comprises a comparison of the time constant of the model used to fit the curve of the model to the at least some of the plurality of performance values for the person with the time constant of the model used to fit the curve of the model to the plurality of performance values for one or more further persons.

10. Apparatus according to any of claims 6-9, wherein the input unit is configured to provide the processing unit with one or more curves of the model each fit to a plurality of performance values for one or more further persons generated from results of a plurality of trials of the test undertaken by the one or more further persons, and wherein the information about the person comprises a comparison of the curve of the model fit to the at least some of the plurality of performance values for the person with the one or more curves of the model each fit to a plurality of performance values for one or more further persons.

11. Apparatus according to any of claims 6-10, wherein the information derived from the curve of the model fit to the at least some of the plurality of performance values comprises an asymptotic performance value of the model used to fit the curve of the model to the at least some of the plurality of performance values, and wherein the information about the person comprises one or more trials of the plurality of trials that were undertaken by the person after a trial that has a performance value within a threshold value of the asymptotic performance value of the model.

12. Apparatus according to any of claims 1-11, wherein the determination of the information about the person comprises the calculation of the curve of the model fit to the at least some of the plurality of performance values and the calculation of performance variability between at least one consecutive pair of performance values, and wherein the processing unit is configured to determine the at least some of the plurality of performance values as the performance values before a performance value that is one of the consecutive pair performance values that have a performance variability equal to or below a threshold value.

13. Apparatus according to any of claims 1-12, wherein the input unit is configured to provide the processing unit with results of a plurality of trials of a second test, wherein the results of each trial of the second test is generated by the person undertaking a plurality of events of each trial of the second test; wherein the processing unit is configured to determine a plurality of performance values for the results of the plurality of trials of the second test, wherein a performance value is determined for each trial of the plurality of trials of the second test, wherein the processing unit is configured to calculate a curve of the model fit to at least some of the plurality of performance values of the second test and/or to calculate performance variability between at least one consecutive pair of performance values of the second test, and wherein the information about the person comprises a comparison of the curve of the model fit to at the least some of the plurality of performance values of the test with the curve of the model fit to at the least some of the plurality of performance values of the second test and/or a comparison of the performance variability between at least one consecutive pair of performance values of the test with the performance variability between at least one consecutive pair of performance values of the second test.

14. A cognitive performance determination method (100), comprising:

providing (110) a processing unit with results of a plurality of trials of a test, wherein the results of each trial is generated by a person undertaking a plurality of events of each trial;
determining (120) by the processing unit a plurality of performance values for the results of the plurality of trials of the test, wherein a performance value is determined for each trial of the plurality of trials; and
determining (130) by the processing unit information about the person, and wherein the determining the information about the person comprises calculating a curve of a model fit to at least some of the plurality of performance values and/or the determining the information about the person comprises calculating performance variability between at least one consecutive pair of performance values.

15. A computer program element for controlling an apparatus according to any of claims 1-13 which when executed by a processor is configured to carry out the method of claim 13.

FIG. 1

**FIG. 2**

**FIG. 3**

tst ~ t | Asym 1.00 tau 0.20 lcr 1.61 err 0.02

**FIG. 4**

tst ~ t I Asym 1.00 tau 0.50 lcr 0.69 err 0.02

FIG. 5

tst ~ t I Asym 1.00 tau 1.00 lcr 0.00 err 0.02

FIG. 6

tst ~ t I Asym 1.00 tau 2.00 lcr - 0.69 err 0.02

FIG. 7

FIG. 8

Europäisches
Patentamt

European
Patent Office

Office européen
des brevets

# EUROPEAN SEARCH REPORT

Application Number

EP 22 17 5179

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/044304 A1 (GROENENDIJK MARTINE [NL] ET AL) 12 February 2015 (2015-02-12) * paragraph [0001] * * paragraph [0098] * * paragraph [0101] * * figures 3,4 * * paragraph [0102] - paragraph [0104] * * paragraph [0239] - paragraph [0240] * ───── | 1-15 | INV. G16H10/20 G16H50/20 |
| X | WO 2016/145372 A1 (AKILI INTERACTIVE LABS INC [US]) 15 September 2016 (2016-09-15) * paragraph [00124] - paragraph [00125] * * paragraph [00166] - paragraph [00167] * * paragraph [0015] * * paragraph [00114] * ───── | 1-15 | |
| X | WO 2018/039610 A1 (AKILI INTERACTIVE LABS INC [US]) 1 March 2018 (2018-03-01) * paragraph [0091] - paragraph [0093] * * paragraph [0177] * * paragraph [0213] - paragraph [0215] * * paragraph [0245] - paragraph [0247] * * paragraph [0303] - paragraph [0304] * * * * paragraph [0333] - paragraph [0334] * ───── | 1-15 | |

TECHNICAL FIELDS
SEARCHED      (IPC)

G16H

The present search report has been drawn up for all claims

| Place of search | Date of completion of the search | Examiner |
|---|---|---|
| Munich | 8 November 2022 | Megalou-Nash, M |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
..............................................................................
& : member of the same patent family, corresponding
document

EPO FORM 1503 03.82 (P04C01)

**EP 4 283 622 A1**

## ANNEX TO THE EUROPEAN SEARCH REPORT
## ON EUROPEAN PATENT APPLICATION NO.

EP 22 17 5179

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2022

| Patent document cited in search report | | | Publication date | Patent family member(s) | | Publication date |
|---|---|---|---|---|---|---|
| US 2015044304 | A1 | | 12-02-2015 | AU 2012331692 A1 | | 19-06-2014 |
| | | | | BR 112014010166 A2 | | 13-06-2017 |
| | | | | CA 2853931 A1 | | 10-05-2013 |
| | | | | CN 104136024 A | | 05-11-2014 |
| | | | | EP 2773339 A1 | | 10-09-2014 |
| | | | | HK 1201744 A1 | | 11-09-2015 |
| | | | | JP 6719860 B2 | | 08-07-2020 |
| | | | | JP 2014532696 A | | 08-12-2014 |
| | | | | RU 2014121888 A | | 10-12-2015 |
| | | | | US 2015044304 A1 | | 12-02-2015 |
| | | | | WO 2013066168 A1 | | 10-05-2013 |
| WO 2016145372 | A1 | | 15-09-2016 | AU 2016228778 A1 | | 28-09-2017 |
| | | | | AU 2021202325 A1 | | 13-05-2021 |
| | | | | CA 2979390 A1 | | 15-09-2016 |
| | | | | EP 3267891 A1 | | 17-01-2018 |
| | | | | JP 2018512202 A | | 17-05-2018 |
| | | | | JP 2021058681 A | | 15-04-2021 |
| | | | | JP 2022097488 A | | 30-06-2022 |
| | | | | KR 20180041089 A | | 23-04-2018 |
| | | | | US 2016262680 A1 | | 15-09-2016 |
| | | | | US 2022117528 A1 | | 21-04-2022 |
| | | | | WO 2016145372 A1 | | 15-09-2016 |
| WO 2018039610 | A1 | | 01-03-2018 | AU 2017314831 A1 | | 14-03-2019 |
| | | | | CA 3035122 A1 | | 01-03-2018 |
| | | | | CN 110022768 A | | 16-07-2019 |
| | | | | EP 3503809 A1 | | 03-07-2019 |
| | | | | JP 7077303 B2 | | 30-05-2022 |
| | | | | JP 2019534061 A | | 28-11-2019 |
| | | | | JP 2022117988 A | | 12-08-2022 |
| | | | | KR 20190067774 A | | 17-06-2019 |
| | | | | US 2019216392 A1 | | 18-07-2019 |
| | | | | US 2022240851 A1 | | 04-08-2022 |
| | | | | WO 2018039610 A1 | | 01-03-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WESNES, K. ; PINCOCK, C.** Practice effects on cognitive tasks: a major problem?. *The Lancet Neurology,* 2002, vol. 1 (8), 473
- **BARTELS, C. ; WEGRZYN, M. ; WIEDL, A. ; ACK-ERMANN, V. ; EHRENREICH, H.** Practice effects in healthy adults: a longitudinal study on frequent repetitive cognitive testing. *BMC neuroscience,* 2010, vol. 11 (1), 1-12
- **JUTTEN, R. J. ; GRANDOIT, E. ; FOLDI, N. S. ; SIKKES, S. A. ; JONES, R. N. ; CHOI, S. E. ; RABIN, L. A.** Lower practice effects as a marker of cognitive performance and dementia risk: a literature review. *Alzheimer's & Dementia: Diagnosis, Assessment & Disease Monitoring,* 2020, vol. 12 (1), e12055

- Modern Applied Statistics With S. **VENABLES, W. N. ; RIPLEY, B. D.** Technometrics. Springer Science+Business Media, 2003, vol. 45
- **S. VERMEENT et al.** Evidence of Validity for a Newly Developed Digital Cognitive Test Battery. *Frontiers in Psychology,* 2020, vol. 11
- **S. VERMEENT et al.** Philips Intellispace Cognition digital test battery: equivalence and measurement invariance compared to traditional analog test versions. *The clinical Neuropsychologist,* 2021